# EUROPÄISCHE PATENTANMELDUNG

(11) **EP 2 599 514 A1**
(43) Veröffentlichungstag der Anmeldung: **05.06.2013**
(21) Anmeldenummer: 11401650.4
(22) Anmeldetag: 01.12.2011
(51) Int. Cl.: A61M 15/06, A61M 11/04, A24F 47/00

(54) **Heißluftextraktionsinhalator mit Inhalationskühlleitung**

(71) Anmelder: Stobi GmbH & Co. KG, 78532 Tuttlingen (DE)
(72) Erfinder: Storz, Markus, 78532 Tuttlingen (DE)
(74) Vertreter: Späth, Dieter

(57) **Zusammenfassung**

Heißluftextraktionsinhalator (1), mit einem Wärmeerzeuger (3) zum Erwärmen eines Luftstroms, der nach dem Wärmeerzeuger (3) eine in Strömungsrichtung dahinter angeordnete Füllkammer (13) für unter Wärmeeinwirkung aerosolbildende Substanzen als Heißluftstrom durchströmt und der als Einatemluft an einem Inhalatorauslass (4) austritt. Der Inhalatorauslass (4) weist eine Inhalationsleitung (9) mit Mundstück (10) auf, an dem der Anwender zur Inhalation saugt. Die Erfindung schlägt vor, die Inhalationsleitung (9) derart auszubilden, dass sie den heißen Luftstrom zur Bildung der Einatemluft vielfach umlenkt und in einer turbulenten Strömung führt. Die turbulente Strömung fördert den Wärmeübergang von dem Heißluftstrom zu einer Innenwand (15) der Inhalationsleitung (9), die vorzugsweise uneben ausgebildet ist und Verwirbelungselemente (18) für den Luftstrom aufweist. Die Außenwand (16) der Inhalationsleitung (9) kann außerdem Kühlrippen (21) aufweisen, um die Wärmeübertragung von der Inhalationsleitung (9) zur Umgebungsluft zu steigern. Durch die vorgeschlagenen Maßnahmen weist die Inhalationsleitung (9) des Heißluftextraktionsinhalators (1), die vorzugsweise als Metallwellschlauch bzw. Metallwellrohr ausgeführt ist, gegenüber herkömmlichen Saugschläuchen oder Saugrohren eine verbesserte Kühlleistung für die Einatemluft auf.

## Beschreibung

Die Erfindung betrifft einen Heißluftextraktionsinhalator, mit einem Wärmeerzeuger zum Erwärmen eines Luftstromes, der nach dem Wärmeerzeuger eine in Strömungsrichtung dahinter angeordnete Füllkammer für unter Wärmeeinwirkung aerosolbildende Substanzen als Heißluftstrom durchströmt und als Einatemluft an einem Inhalatorauslass austritt, wobei der Inhalatorauslass eine Inhalationsleitung mit Mundstück aufweist.

Derartige therapeutische Verdampfungsvorrichtungen zur Erzeugung von Aroma- und/oder Wirkstoffdämpfen für die Inhalation sind aus dem Stand der Technik in vielfältigen Ausführungsformen bekannt. Beispielhaft wird auf die Patentschriften DE 100 42 396 B4 und DE 198 03 376 C1 verwiesen. Die dort beschriebenen Inhalatoren dienen zur thermischen Verdampfung und anschließenden Inhalation von verdampfbaren, also unter Wärmeeinwirkung aerosolbildenden Substanzen. Einfache Heißluftextraktionsinhalatoren verfügen lediglich über eine beheizte Füllkammer zur Erwärmung der zu verdampfenden Substanzen. Diese Bauweise ermöglicht eine nur geringe Verdampfung, da während der Inhalation die nachströmende unbeheizte Luft den Verdampfungsvorgang wieder unterbricht. Es muss dann gewartet werden bis die Strahlungswärme der Füllkammer die enthaltenen Substanzen wieder für die Verdampfung hinreichend erwärmt hat. Besondere Maßnahmen zur Kühlung der Inhalationsluft sind bei dieser Bauweise aufgrund der geringen Heizleistung nicht erforderlich.

Wesentlich ergiebiger ist die Methode, bei der die Substanzen mit einem heißem Luftstrom beaufschlagt, werden, der beim Passieren eines Wärmeerzeugers aufgeheizt wird. Die Heißluft durchströmt die Füllkammer mit den Substanzen, wobei die Substanzen ansich verdampfen oder ihre Wirkstoffe ausdünsten (extrahieren), die in die Heißluft übergehen. Diese zum Beispiel mit Aromen und/oder Wirkstoffen befrachtete Heißluft wird als Einatemluft inhaliert, nachdem sie auf eine zum Einatmen angenehme Temperatur heruntergekühlt wurde, wobei die Wirkstoffe nach dem Inhalieren über die Lunge in den Blutkreislauf gelangen. Für die Heißluftextraktion werden häufig Heilkräuter oder andere geeignete pflanzliche Substanzen verwendet, die dem Verwendungszweck angemessen zerkleinert sind oder aber auch synthetische Substanzen mit therapeutischen Wirkstoffen, die in pulverisierter Form vorliegen. Es können auch flüssige Substanzen verdampft werden. Zur Aerosolbildung müssen diese Substanzen mit Heißluft von beispielsweise 200° C beaufschlagt werden.

Bei der effizienteren Methode der Lufterwärmung wird üblicherweise ein Wärmeerzeuger verwendet, der mindestens einen Luftkanal für den zu erwärmenden Luftstrom aufweist. Der Luftkanal kann den Wärmeerzeuger innen durchsetzen oder außen einschließen. Prinzipiell gibt es bei bekannten gattungsgemäßen Heißluftextraktionsinhalatoren zwei Grundprinzipien zur Erzeugung des heißen Luftstroms mittels des Wärmeerzeugers. Zum einen kann der Luftstrom durch Ansaugen von Luft an einer Austrittsöffnung oder zum anderen durch Einblasen von Luft in eine Eintrittsöffnung des Luftkanals erzeugt werden, wobei der erforderliche Über- bzw. Unterdruck mittels Lungenkraft oder unter Verwendung eines Gebläses oder einer Pumpe, beispielsweise einer Membranpumpe, aufgebaut werden kann. Durch den thermischen Kontakt mit dem Wärmeerzeuger wird die an einem Inhalatoreinlass in den Luftkanal einströmende Umgebungsluft in Abhängigkeit von der von dem Wärmeerzeuger ausgehenden Temperatur und dem Volumenstrom durch den Luftkanal von Zimmertemperatur auf beispielsweise 200° C erwärmt. Die Temperatur ist abgestimmt auf den Siede- oder Extraktionspunkt der für die Inhalation verwendeten aerosolbildenden Substanzen, so dass deren Wirkstoffe und/oder Aromen beim Durchtritt des Heißluftstroms durch die Füllkammer verdampfen und die Aerosole von dem Luftstrom aufgenommen werden. Dabei sinkt die Temperatur der Heißluft etwas ab.

Nach dem Passieren der Füllkammer mit den aerosolbildenden Substanzen steht der aerosolbefrachtete Luftstrom als Einatemluft zur Inhalation zur Verfügung. Bei einer effizienten Verdampfung weist die Einatemluft jedoch eine zum Einatmen viel zu hohe Temperatur auf. Sie muss vor dem Einatmen auf eine zum Einatmen geeignete und für den Anwender des Inhalators angenehme Temperatur gekühlt werden. Dies geschieht im Bereich eines Inhalatorauslasses des Heißluftextraktionsinhalators, der in Strömungsrichtung nach der Füllkammer angeordnet ist und zum Aufnehmen der Atemluft über den Mund eine Inhalationsleitung mit anschließendem Mundstück aufweist.

Das Problem einer definierten Abkühlung der Einatemluft ist naturgemäß einfacher zu lösen, wenn die Temperatur des heißen Luftstroms nach dem Durchtritt durch die in die Füllkammer eingebrachten aerosolbildenden Substanzen während der gesamten Anwendungdauer des Heißluftextraktionsinhalators annähernd konstant ist. Eine sich nur geringfügig ändernde Temperatur des Luftstroms nach der Füllkammer setzt voraus, dass der Volumenstrom in dem Luftkanal nicht zu stark variiert. Dies kann sichergestellt werden, indem der Luftstrom mittels einer dem Luftkanal des Wärmeerzeugers vorgeschalteten Membranpumpe erzeugt wird, oder falls der Luftstrom allein durch Atmung erzeugt wird, indem spezielle mechanische Einrichtungen zur Begrenzung des Volumenstroms vorgesehen werden. Zusätzlich ist es noch von Vorteil, die von dem Wärmeerzeuger ausgehende Temperatur unter Verwendung einer geeigneten Regeltechnik präzise gleichbleibend auf die Verdampfungs- oder Extraktionstemperatur zu regeln. Weiter soll die Dimension der Inhalationsleitung für eine kompakte Bauweise möglichst gering gehalten werden. Ausgehend von dem Stand der Technik liegt der Erfindung die Aufgabe zugrunde, eine Möglichkeit vorzuschlagen, die Kühlleistung der Inhalationsleitung deutlich zu steigern, insbesondere zu vervielfachen, um die Einatemluft in jedem Fall, d.h. auch bei einer Variation des Volumenstroms durch den Heißluftextraktionsinhalator soweit zuverlässig auf eine für die Einatmung geeignete Temperatur abzukühlen, so dass eine Verletzung des Anwenders ausgeschlossen ist und dabei eine möglichst kompakte Bauweise realisiert werden kann.

Diese Aufgabe wird erfindungsgemäß durch einen Heißluftexktrationsinhalator mit den Merkmalen des Anspruchs 1 gelöst. Weitere vorteilhafte Ausgestaltungen sind den Unteransprüchen zu entnehmen.

Kerngedanke der Erfindung ist es, den Wärmeübergang von der Einatemluft zu der Inhalationsleitung sowie den Wärmeübergang von der Inhalationsleitung zu der Umgebungsluft deutlich zu verbessern, so dass die Inhalationsleitung möglichst gering dimensioniert werden kann.

Danach weist der erfindungsgemäße Heißluftexktrationsinhalator eine Inhalationsleitung auf, die den heißen Luftstrom, der aus der Füllkammer austritt, zum Gebrauch als Einatemluft mit reduzierter Temperatur vielfach umlenkt und in einer turbulenten Strömung führt. Dazu weist die Inhalationsleitung, die als Schlauch oder Rohr ausgeführt sein kann, vorzugsweise in einer Ausführungsform eine unebene Innenwand und in einer anderen bevorzugten Ausführungsform an der Innenwand bzw. in der Inhalationsleitung angeordnete Verwirbelungselemente auf. Die Innenwand kann glatt oder rau sein.

Die verstärkte Durchmischung der Gas- und Flüssigkeitsteilchen der Einatemluft aufgrund der von Turbulenzen verursachten Fluktuationsbewegung ist eine der wichtigsten Eigenschaften der in der Inhalationsleitung erzeugten turbulenten Strömung. Dabei kann die Bewegung der vorstehend genannten Fluidteilchen in allen Größenskalen auftreten, wobei sich die turbulente Strömungsform durch eine dreidimensionale, scheinbar zufällige Bewegung der Fluidteilchen von der laminaren Strömung abhebt. Des weiteren begünstigt die turbulente Querströmung der Fluidteilchen den Wärmetransport in Strömungsrichtung zu der Innenwand der Inhalationsleitung, so dass die Temperaturverluste in der Strömung des Strömungskanals steigen. Zudem wird durch die Querströmung der Fluidteilchen, der sich bei einer laminaren Strömung quer zu der Richtung des Strömungskanals ausbildenden Temperaturgradient, bei der turbulenten Strömung vermieden oder stark reduziert. Durch die fortlaufende Vermischung der Fluidteilchen weist die Einatemluft eine im wesentlichen homogene Temperaturverteilung über den Querschnitt des Strömungskanals auf.

Die Inhalationsleitung führt Wärme an die Umgebungsluft ab, die Einatemluft wird gekühlt. Am Mundstück ist die Temperatur der Einatemluft deutlich unter der Temperatur des Heißluftstroms in der Füllkammer, der heiße Luftstrom kann in der Inhalationsleitung effektiv auf eine für den Anwender des Heißluftextraktionsinhalators angenehme Temperatur gekühlt werden. Die Inhalationsleitung kann an sich eine beliebige Querschnittsform aufweisen und sich in Längsrichtung gerade oder gewunden erstrecken. Sie kann zudem in Strömungsrichtung der Einatemluft ein- oder mehrfach abgeknickt bzw. gebogen sein. Durch einen solchen nicht geraden Verlauf gelangen die Fluidteilchen des heißen Luftstroms an den Knick- oder Biegestellen an die Innenwand der Inhalationsleitung. Dies führt zu erheblichen Turbulenzen in der Einatemluft die durch eine raue Innenwand und/oder durch gegebenenfalls vorgesehene Verwirbelungselemente zusätzlich verstärkt werden.

Besonders vorteilhaft sind Verwirbelungselemente, die zumindest teilweise quer oder schräg zu der jeweiligen Erstreckungsrichtung der Inhalationsleitung verlaufen. Die Verwirbelungselemente sind idealerweise an die Innenwand als Rippen oder dergleichen angeformt, die sich dort vorzugsweise als Erhebungen kreisförmig umlaufend, beabstandet voneinander erstrecken und gegenüber den Erhebungen vertiefte Zwischenräume bestimmen. Die Innenwand weist damit in Längsrichtung eine Vielzahl von Erhebungen und Vertiefungen in Folge auf, die sich an der Außenwand deckend oder versetzt zueinander fortsetzen können. Eine derartige Innen- und Außenwand weist beispielsweise ein Wellrohr oder Wellschlauch auf, die sich im wesentlichen durch ihre Wandstärke und ihre Verformungseigenschaften voneinander unterscheiden.

Bei einer bevorzugten Ausführungsform der Erfindung ist die Inhalationsleitung zusätzlich zu einer Wendel mit einer oder mehreren Windungen geformt. Die Wendel kann beidseitig gestreckte Endabschnitte aufweisen. Wesentlich ist dabei, dass die einzelnen Windungen der Wendel ihre Lage zueinander während des Gebrauchs des Heißluftextraktionsinhalators beibehalten. Die Windungen der Wendel sind derart ausgebildet, dass eine fortlaufende günstige Verwirbelung der Einatemluft an der Innenwand mit großen Turbulenzen erreicht wird. Um eine Streckung der Wendel durch den Anwender bei der Benutzung des Heißluftextraktionsinhalators zu vermeiden, die eine Minderung der Kühlleistung der Inhalationsleitung nach sich zieht, ist die Wendel bei einer bevorzugten Ausführungsform der Erfindung biegesteif und nur plastisch verformbar als gewendeltes Inhalationsrohr ausgeführt. Eine derartige Ausführungsform vervielfacht die Kühlleistung der Inhalationsleitung.

Dem gegenüber bietet eine als Inhalationsschlauch ausgeführte Inhalationsleitung aufgrund der elastischen Verformbarkeit mehr Benutzungskomfort für den Anwender. Durch die Beweglichkeit der Inhalationsleitung in diesem Fall kann der Anwender die Kopfhaltung verändern, ohne die Ausrichtung des Heißluftextraktionsinhalators anpassen zu müssen. Dieser Komfort wird jedoch durch eine geringere Kühlleistung bei unsachgemäßer Verformung der Inhalationsleitung erkauft.

Prinzipiell kann die vorgeschlagene Inhalationsleitung bei jeder Art von Heißluftextraktionsinhalatoren verwendet werden, unabhängig davon, ob es sich um ein Tisch- oder Standgerät oder ein Handgerät handelt. Dabei ist es unerheblich, ob der Wärmeerzeuger zum Erwärmen des Luftstroms elektrisch- oder gas- oder in sonstiger Weise beheizt ist und ob der Wärmeerzeuger mit einem Wärmetauscher verbunden ist, den der Luftstrom zum Erhitzen durchströmt. Auch der innere Aufbau des Heißluftextraktionsinhalators ist an sich beliebig, solange der Heißluftstrom nach dem Durchströmen der Füllkammer als heiße Einatemluft an einem Inhalatorauslass ansteht und vor dem Abkühlen auf eine annehmbare Temperatur heruntergekühlt werden muss. Auch das Material aus dem die Inhalationsleitung hergestellt ist, ist an sich frei wählbar, vorausgesetzt, dass es eine gute Wärmeleitfähigkeit aufweist. Als robustes und einfach zu bearbeitendes Material eignet sich ein metallischer Werkstoff besonders für die Inhalationsleitung.

Zur Verbesserung der Wärmeübertragung von der Inhalationsleitung zu der umgebenden Atmosphäre und um eine störende und damit unerwünschte übermäßige Aufheizung der Inhalationsleitung zu verhindern, die eine verminderte Kühlleistung nach sich ziehen würde, kann die Außenwand der Inhalationsleitung des erfindungsgemäßen Heißluftextraktionsinhalators ähnlich der Innenwand der Inhalationsleitung ausgebildet sein. Dies führt zu einer Vergrößerung der Oberfläche und damit zu einer Vergrößerung der Kühlleistung. Vorzugsweise weist die Inhalationsleitung des erfindungsgemäßen Heißluftextraktionsinhalators eine unebene Außenwand auf, die ähnlich der unebenen Innenwand ausgebildet ist. Dabei kann die Außenwand glatt oder rau ausgebildet sein und Kühlrippen zur Vergrößerung der äußeren Oberfläche aufweisen. Die Gestaltung der Außenwand der Inhalationsleitung kann unabhängig vom Verlauf und/oder der Ausgestaltung der Innenwand oder etwaiger Einbauten erfolgen. Eine Kombination der aufgezeigten Maßnahmen verbessert die Kühlleistung.

Nachfolgend wird die Erfindung anhand eines in der Zeichnung dargestellten Ausführungsbeispiels näher erläutert. Weitere Merkmale der Erfindung ergeben sich aus der folgenden Beschreibung des Ausführungsbeispiels der Erfindung in Verbindung mit den Ansprüchen und der beigefügten Zeichnung. Die einzelnen Merkmale der Erfindung können für sich allein oder zu mehreren bei unterschiedlichen Ausführungsformen der Erfindung verwirklicht sein. Es zeigen:
- Figur 1: einen erfindungsgemäßen Heißluftextraktionsinhalator mit einer sich gerade erstreckten Inhalationsleitung am Inhalatorauslass, in einer Längsschnittdarstellung;
- Figur 2: eine Variante der Inhalationsleitung aus Figur 1, ausgeführt als gestreckter Wellschlauch, in einer Achsschnittdarstellung; und
- Figur 3: eine weitere Variante der Inhalationsleitung aus Figur 1, ausgeführt als gewendeltes Wellrohr, in perspektivischer Darstellung.

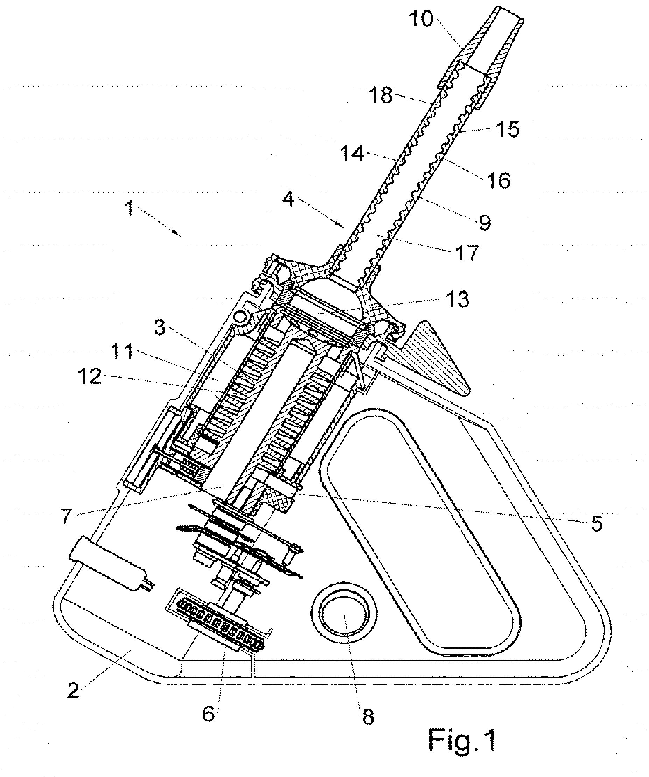

Die Figur 1 zeigt ein erstes Ausführungsbeispiel eines erfindungsgemäßen Heißluftextraktionsinhalators 1. Der Heißluftextraktionsinhalator 1 weist ein Inhalatorgehäuse 2 auf, in dem ein zu dem Inhalatorgehäuse 2 hin wärmeisolierter Wärmeerzeuger 3 zum Erwärmen eines Luftstroms angeordnet ist, der bei Beaufschlagung eines Inhalatorauslasses 4 des Heißluftextraktionsinhalators 1 mit Unterdruck am Inhalatoreinlass 5 in den Wärmeerzeuger 3 eintritt und nach dem Passieren des Wärmeerzeugers 3 an dem Inhalatorauslass 4 als Heißluftstrom austritt. Der Wärmeerzeuger 3 ist elektrisch beheizt, er weist eine elektrische Heizpatrone 7 auf, wobei dessen Temperatur mit einem Temperatureinsteller 6 wählbar ist und eine Versorgungsspannung des Wärmerzeugers 3 über einen Netzspannungsschalter 8 schaltbar ist.

Der Heißluftextraktionsinhalator 1 ist als Handgerät ausgebildet. Er weist als Inhalatorauslass 4 eine Inhalationsleitung 9 mit abnehmbarem Mundstück 10 auf. Der Wärmeerzeuger 3 ist von einem isolierten Mantelrohr 11 umgeben und weist zwei um die Heizpatrone 7 als Doppelhelix ausgebildete umlaufende Luftkanäle 12 auf. An die Luftkanäle 12 schließt eine Füllkammer 13 zur Aufnahme von unter Wärmeeinwirkung aerosolbildenden Substanzen an. Durch Saugen an dem Mundstück 10 des Inhalatorauslasses 4 kann ein Anwender des Heißluftextraktionsinhalators 1 einen Luftstrom durch die Luftkanäle 12 des Wärmetauschers 3 bewirken. Der Luftstrom wird beim Passieren des Wärmetauschers 3 erhitzt und durchströmt die Füllkammer 13 als Heißluftstrom mit einer Temperatur von beispielsweise 200 bis 235 °C, bevor er zu dem Inhalatorauslass 4 gelangt. Der Heißluftstrom extrahiert in der zwischen dem Wärmeerzeuger 3 und der Inhalationsleitung 9 angeordneten Füllkammer 12, welche die aerosolbildenden Substanzen für den Inhalationsvorgang aufnimmt, Aromen und/oder Wirkstoffe durch Verdampfen oder Vergasen (Extraktion) aus diesen Substanzen.

Der mit Aerosolen befrachtete Heißluftstrom tritt als heiße Einatemluft in die Inhalationsleitung 9 ein und wird auf dem Weg zum Mundstück 10 an einer Wandung 14 der Inhalationsleitung 9 auf eine zum Einatmen geeignete Temperatur abgekühlt. Für die Inhalationsleitung 9 ist ein gut wärmeleitendes Material, beispielsweise Metall, gewählt. Sie weist eine Innenwand 15, eine Außenwand 16 und einen von der Wandung 14 begrenzten Strömungskanal 17 auf. Die in der Figur 1 dargestellte Ausführungsform weist eine sich im wesentlichen gerade zwischen der Füllkammer 13 und dem Mundstück 10 erstreckende Inhalationsleitung 9 auf. Die Außenwand 16 ist dabei glatt und eben ausgebildet, während an der Innenwand 15 Verwirbelungselemente 18 angeordnet sind, so dass die Innenwand 15 zwar glatt, jedoch uneben ausgebildet ist. Die Verwirbelungselemente 18 lenken die heiße Einatemluft in der Inhalationsleitung 9 vielfach um und verursachen Wirbel in der Einatemluft, so dass die Einatemluft in einer turbulenten Strömung durch die Inhalationsleitung 9 geführt wird. Durch die turbulente Strömung weist die strömende Einatemluft Geschwindigkeitskomponenten in axialer und in radialer Richtung der Inhalationsleitung 9 auf. Die radiale Geschwindigkeitskomponente verbessert den Wärmeübergang von der strömenden heißen Einatemluft zu der Innenwand 15 der Wandung 14 der Inhalationsleitung 9 und steigert die Kühlleistung des Inhalatorauslasses 4.

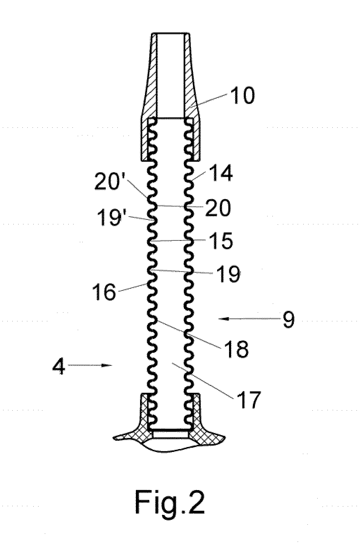

Figur 2 zeigt eine Variante der Inhalationsleitung 9 aus Figur 1, ebenfalls in einer Achsschnittdarstellung. Die Inhalationsleitung 9 weist zudem wie vorstehend beschrieben eine Wandung 14 mit einer Außenwand 16 und einer Innenwand 15 sowie einen von der Wandung 14 begrenzten Strömungskanal 17 für die Einatemluft auf. Die Innenwand 15 und die Außenwand 16 sind glatt und uneben ausgebildet und weisen insbesondere in radialer Richtung Vertiefungen 19 bzw. 19' und Erhöhungen 20 bzw. 20' auf, die in axialer Richtung wellenförmig aufeinanderfolgen. Für die Inhalationsleitung 9 ist bei diesem Ausführungsbeispiel ein Metallwellschlauch verwendet. Die Erhöhungen 20 der Innenwand 15 bilden für den Strömungskanal 17 die Verwirbelungselemente 18, die die heiße Einatemluft in eine turbulente Strömung versetzen. Die Erhöhungen 20 an der Außenwand 16 bilden Kühlrippen 21 der Inhalationsleitung 9 des Inhalatorauslasses 4. Die Kühlrippen 21 vergrößern für den Wärmeaustausch mit der Umgebungsluft die wirksame Fläche der Außenwand 16. Durch die Kombination der Verwirbelungselemente 18 mit den Kühlrippen 21 an dem Inhalatorauslass 4 wird eine höhere Kühlleistung der Inhalationsleitung 9 erreicht.

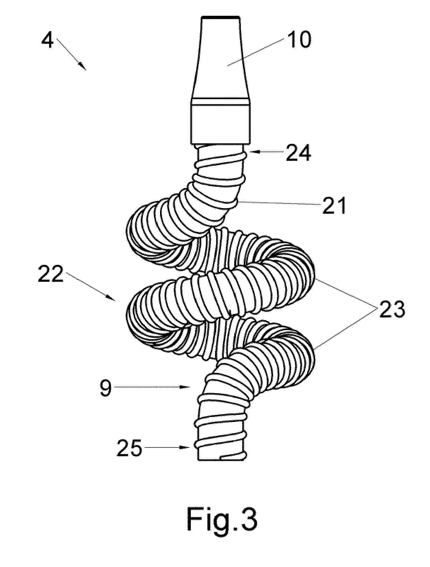

Figur 3 zeigt eine zweite Variante des Inhalatorauslasses 4 aus Figur 1 in perspektivischer Darstellung. Die Inhalationsleitung 9 entspricht im Aufbau im wesentlichen dem Metallwellschlauch aus Figur 2. Sie unterscheidet sich von diesem zum einen durch eine Wandstärke der Wandung 14 und zum anderen dadurch, dass sie nicht gerade ist, sondern eine Wendel 22 mit mehreren Windungen 23 aufweist, die zwischen zwei gestreckten Endabschnitten 24, 25 angeordnet ist. An einem in Strömungsrichtung der Einatemluft hinterer Endabschnitt 24 ist das Mundstück 10 angeordnet, ein vorderer Endabschnitt 25 ist mit der in dieser Figur nicht dargestellten Füllkammer 13 des Heißluftextraktionsinhalators 1 verbunden. Die Wendel 22 erstreckt sich über den größten Teil der Länge der als Metallwellrohr ausgebildeten Inhalationsleitung 9, die insbesondere biegesteif und plastisch nur schwer verformbar ausgeführt ist. Das Metallwellrohr 9 behält bei sachgemäßem Umgang seine vorgegebene Form unverändert bei. Die Inhalationsleitung 9 weist außerdem an der Außenwand 16 Kühlrippen 21 und an der Innenwand 15 Verwirbelungselemente 18 auf, die in Umfangsrichtung umlaufend verlaufen. Die Innenwand 15 mit den dort angeordneten Verwirbelungselementen 18 ist in der Figur 3 nicht sichtbar. Es hat sich überraschend gezeigt, dass die Wendelform oder allgemein eine nicht gerade Kurvenform, die die Richtung des Einatemluftstroms permanent oder jedenfalls oft ändert, die Kühlleistung der Inhalationsleitung 9 vervielfacht.

## Patentansprüche

1. Heißluftextraktionsinhalator (1), mit einem Wärmeerzeuger (3) zum Erwärmen eines Luftstroms, der nach dem Wärmeerzeuger (3) eine in Strömungsrichtung dahinter angeordnete Füllkammer (13) für unter Wärmeeinwirkung aerosolbildende Substanzen als Heißluftstrom durchströmt und als Einatemluft an einem Inhalatorauslass (4) austritt, wobei der Inhalatorauslass (4) eine Inhalationsleitung (9) mit Mundstück (10) aufweist, **dadurch gekennzeichnet, dass** die Inhalationsleitung (9) den Luftstrom vielfach umlenkt und in einer turbulenten Strömung führt.

2. Heißluftextraktionsinhalator nach Anspruch 1, **dadurch gekennzeichnet, dass** die Inhalationsleitung (9) eine unebene Innenwand (15) aufweist.

3. Heißluftextraktionsinhalator nach Anspruch 2, **dadurch gekennzeichnet, dass** die Innenwand (15) Verwirbelungselemente (18) für den Luftstrom aufweist.

4. Heißluftextraktionsinhalator nach einem der vorstehenden Ansprüche, **dadurch gekennzeichnet, dass** die Inhalationsleitung (9) eine unebene Außenwand (16) aufweist.

5. Heißluftextraktionsinhalator nach Anspruch 4, **dadurch gekennzeichnet, dass** die Außenwand (16) Kühlrippen (21) aufweist.

6. Heißluftextraktionsinhalator nach einem der vorstehenden Ansprüche, **dadurch gekennzeichnet, dass** die Inhalationsleitung (9) als Inhalationsschlauch elastisch verformbar oder als Inhalationsrohr biegesteif und plastisch verformbar ausgeführt ist.

7. Heißluftextraktionsinhalator nach einem der vorstehenden Ansprüche, **dadurch gekennzeichnet, dass** die Inhalationsleitung (9) ein Wellschlauch oder ein Wellrohr ist.

8. Heißluftextraktionsinhalator nach einem der vorstehenden Ansprüche, **dadurch gekennzeichnet, dass** die Inhalationsleitung (9) zumindest über einen Teil ihrer Länge zu einer Wendel (22), Spirale, Schlange, oder als U geformt ist.
